# EUROPÄISCHE PATENTSCHRIFT

(11) **EP 0 628 299 B1**
(45) Veröffentlichungstag und Bekanntmachung des Hinweises auf die Patenterteilung: **30.07.1997**
(21) Anmeldenummer: 94108356.0
(22) Anmeldetag: 31.05.1994
(51) Int. Cl.: A61F 13/06

(54) **Sprunggelenksbandage**
Ankle bandage
Bandage de cheville

(30) Priorität: 05.06.1993 DE 4318791
(43) Veröffentlichungstag der Anmeldung: 14.12.1994
(73) Patentinhaber: SCHÜTT & GRUNDEI ORTHOPÄDIETECHNIK GmbH, D-23556 Lübeck (DE)
(72) Erfinder: Grundei, Hans, Dr., D-23558 Lübeck (DE); Etspüler, Rolf, Dr., D-23562 Lübeck (DE); Schmelzer, Ulrich, Dr., D-23568 Lübeck (DE)
(74) Vertreter: Fuchs, Luderschmidt & Partner Patentanwälte

(56) Entgegenhaltungen:
- DE-C- 3 441 496
- DE-U- 9 112 765
- FR-A- 2 606 630
- FR-A- 2 613 932
- US-A- 4 729 370

## Beschreibung

Die Erfindung betrifft eine Sprunggelenksbandage mit den Merkmalen des Oberbegriffs des Anspruchs 1, wie sie beispielsweise bekannt sind aus der DE 91 12 765 U1 oder der DE 34 41 496 C1. Derartige Bandagen kommen zum Einsatz bei einer Außenbandsläsion oder gar einem Außenbandsriß. Vor der Anwendung der bekannten Bandagen war es meist üblich, den Fuß beispielsweise durch einen Gipsverband ruhig zu stellen, solange, bis das Außenband wieder verheilt war. Wichtig dabei ist, daß der Fuß weitgehend ruhiggestellt werden muß, da sonst eine neue eventuelle Suppination die Teilbehandlungserfolge wieder zunichte macht. Unter Suppination versteht man im übrigen das Nachinnenknicken des Fußes. Ein Behandlungserfolg durch eine erneute Suppination wird dadurch zunichte gemacht, daß das gerade heilende Band unter Zug gesetzt wird.

Im einzelnen bestehen die aus den oben näher bezeichneten Druckschriften bekannten Sprunggelenksbandagen aus einem socken- oder strumpfförmigen Grundkörper, der sowohl einen Unterschenkelabschnitt als auch einen Fußabschnitt umfaßt. Es ist ein sogenannter Pronationszügel vorgesehen, mittels dessen zur Entlastung des lädierten Außenbandes der Fuß in eine provozierte Pronationsstellung gebracht wird, damit das Band entlastet ist und eine Suppination eben aufgrund des gespannten Pronationszügels unwahrscheinlich wird. Es sei an dieser Stelle angemerkt, daß man unter Pronation das Gegenteil zu der bereits oben erläuterten Suppination versteht.

Wenn dieses Ziel mit den bekannten Sprunggelenksbandagen auch erreichbar ist, so scheitert deren Einsatz in der Praxis daran, daß die strumpf- oder sockenförmigen Grundkörper über den Fuß und den unteren Unterschenkel gestreift werden müssen, was bei den Indikationsfällen, in denen derartige Bandagen zum Einsatz kommen sollen, für den Patienten mit unerträglichen Schmerzen verbunden ist.

Aus der FR-A-2 613 932 ist eine Fußgelenksbandage bekannt, welche über einen im wesentlichen röhrenförmig ausgebildeten Fußabschnitt verfügt und deren Unterschenkelabschnitt aus zwei zur Überlappung zu bringenden Laschen besteht. Diese Fußgelenksbandage kann einfach über den Fuß gezogen werden. Zum Anlegen werden die sich überlappenden Teile um den Unterschenkel geschlagen, so daß die Fußgelenksbandage sitzt. Allerdings weist diese Fußgelenksbandage kein Pronationszügel auf. Auch ist ihre Stabilität nach dem Anliegen ausschließlich materialbedingt gegeben. Besteht die Bandage aus einem relativ angenehm zu tragenden Material, ist die Stabilität relativ gering. Zur Therapie einer Außenbandsläsion oder gar eines Außenbandsrisses ist eine solche Bandage nicht heranzuziehen. Aber auch die Verwendung eines relativ steifen und unangenehm zu tragenden Materials bringt nicht die Vorteile, welche man sich von einer Pronationsbandage erhofft.

Aufgabe der vorliegenden Erfindung vor diesem Hintergrund ist es, eine Sprunggelenksbandage anzugeben, die einfach anzulegen ist und dennoch bei einem guten Behandlungserfolg größtmögliche Bewegungsfreiheit gestattet, wobei die Stabilität der angelegten Sprunggelenksbandage gewährleistet sein soll.

Die Aufgabe wird gelöst durch eine Sprunggelenksbandage gemäß dem Anspruch 1. Vorteilhafte Weiterbildungen ergeben sich aus den Unteransprüchen.

Demgemäß ist vorgesehen, daß der Fußabschnitt im wesentlichen röhrenförmig ausgebildet ist und der Unterschenkelabschnitt gebildet wird aus zwei teilweise zur Überlappung bringbaren Laschen, die mittels eines Schnellverschlusses in eine röhrenförmige Form, den unteren Unterschenkel umfassend, bringbar sind und daß eine Versteifungseinlage medial in einer Textiltasche liegt, die vom Fersenbereich des Fußabschnittes bis zum Oberrand des Unterschenkelabschnittes reicht. Die Versteifungseinlage kann beispielsweise aus elastischem Kunststoff bestehen.

Wie bei einer - pronationszügellosen - Sprunggelenksbandage gemäß der FR-A-2 613 932 zieht der Patient bei der erfindungsgemäßen Bandage lediglich den Fußabschnitt einfach über seinen Fuß, wonach die Laschen um den Unterschenkel geschlagen werden und der so gebildete Unterschenkelabschnitt einfach durch Betätigung des Schnellverschlusses an den beiden Laschen röhrenförmig fixiert wird.

Gemäß einer vorteilhaften Weiterbildung ist bei der Sprunggelenksbandage medial im Fersenbereich des Fußabschnittes ein elastischer Gurt angebracht. Dieser kann um den Fußabschnitt und den Unterschenkelabschnitt der Bandage geschlungen werden und unter Zugspannung mit seinem freien Ende mittels eines Schnellverschlusses, vorzugsweise eines Klettenverschlusses, arretiert werden. Dieser elastische Gurt dient der Sicherheit der Bandage, indem er den lateralen Pronationszügel an den Grundkörper der Bandage anpreßt.

Die erfindungsgemäße Sprunggelenksbandage besteht vorzugsweise aus atmungsaktivem, elastischem Material. Sie gestattet es, daß der Patient sich weitgehend ungestört bewegen kann trotz der erlittenen Außenbandsläsion bzw. -risses.

Anhand der Zeichnungsfiguren wird die Bandage näher erläutert. Hierbei zeigt:
**Figur 1** die erfindungsgemäße Bandage im angelegten Zustand an einem rechten Fuß, von vom gesehen,
**Figur 2** die Seitenansicht des Fußes mit angelegter Bandage, und
**Figur 3** wie die Bandage angelegt wird.

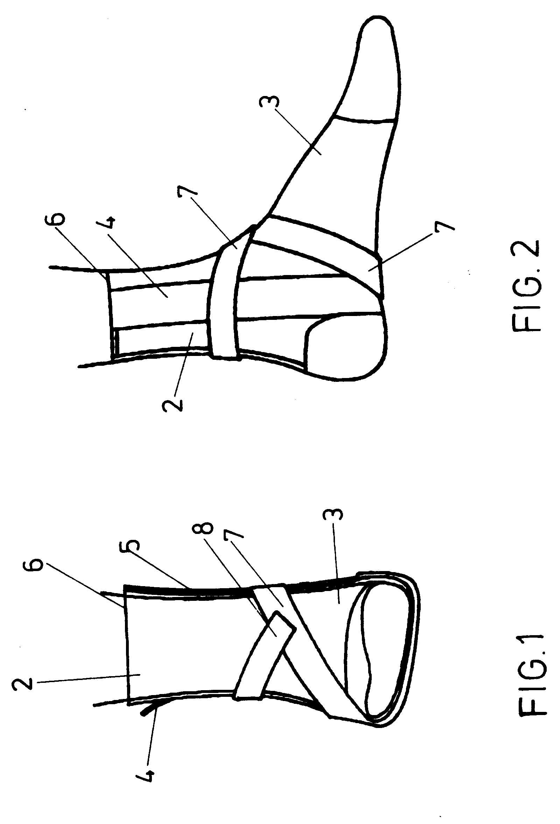

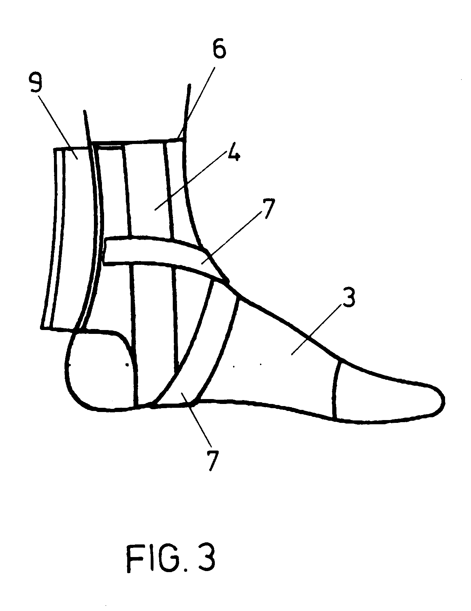

Die Sprunggelenksbandage besteht aus einem Unterschenkelabschnitt 2 und einem Fußabschnitt 3. Im dargestellten Ausführungsbeispiel (Figur 2) wird deutlich, daß die Ferse frei bleibt vom elastischen Material der Bandage. Der Fußabschnitt 3 ist röhrenförmig ausgebildet. Der Patient zieht den Fußabschnitt 3 also einfach über seinen Fuß. Der Unterschenkelabschnitt 2 ist gebildet aus zwei Laschen 9 aus dem elastischen Material (Figur 3), die um den Unterschenkel geschlagen werden und miteinander mit einem an innen befestigten Schnellverschluß verbunden werden.

Figur 2 ist eine laterale Seitenansicht auf einen Fuß mit angelegter Bandage. Hieraus wird deutlich, daß lateral ein Pronationszügel im Fersenbereich angebracht ist. Der Pronationszügel 4 besteht aus einem reiß- und zugfesten Material. Im angelegten Zustand verläuft dieser Zügel 4 lateral im wesentlichen über das Sprunggelenk in Richtung auf den Unterschenkelabschnitt 2. Beim Anlegen wird der Pronationszügel 4 gespannt und der Fuß in die provozierte Pronationsstellung gezwungen (Figur 1). In gespanntem Zustand wird der Pronationszügel 4 mittels eines Schnellverschlusses im oberen Bereich des Unterschenkelabschnittes 2, vorzugsweise mittels eines Klettenverschlusses, an diesem Abschnitt befestigt. Auf der gegenüberliegenden Seite, d.h. medial, ist in einer Textiltasche eine Versteifungseinlage vorgesehen, die im wesentlichen vom Fersenbereich des Fußabschnittes 3 bis zum Oberrand 6 des Unterschenkelabschnittes 2 reicht (vgl. Figur 1).

Im Fersenbereich des Fußabschnittes 3 ist mit seinem einen Ende ein elastischer Gurt 7 angebracht, der zur Erhöhung der Sicherheit der Bandage um den Fußabschnitt 3 und den Unterschenkelabschnitt 2 herumgelegt ist und mit seinem freien Ende schließlich arretiert ist, vorliegend mittels eines Schnellverschlusses zwischen dem freien Ende 8 und der Oberseite des Gurtes 7. Dieser Gurt 7 dient dazu, den Pronationszügel 4 an den Fuß bzw. das Bein anzupressen.

## Patentansprüche

1. Sprunggelenksbandage aus elastischem Material mit einem Unterschenkelabschnitt (2) und einem Fußabschnitt (3), bei der im Fersenbereich des Fußabschnittes (3) ein Pronationszügel (4) aus reiß- und zugfestem Material angebracht ist, der im angelegten Zustand der Bandage lateral im wesentlichen über das Sprunggelenk verlaufend in Richtung auf den Unterschenkelabschnitt (2) spannbar und daran befestigbar ist, dadurch gekennzeichnet, daß der Fußabschnitt (3) im wesentlichen röhrenförmig ausgebildet ist und der Unterschenkelabschnitt (2) gebildet wird aus zwei teilweise zur Überlappung bringbaren Laschen (9), die mittels eines Schnellverschlusses in eine röhrenförmige Form, den unteren Unterschenkel umfassend, bringbar sind und daß medial in einer Textiltasche eine Versteifungseinlage (5) liegt, die vom Fersenbereich des Fußabschnittes (3) bis zum Oberrand (6) des Unterschenkelabschnittes (2) reicht.

2. Sprunggelenksbandage nach Anspruch 1, bei der medial im Fersenbereich des Fußabschnittes (3) ein elastischer Gurt (7) angebracht ist, der in einer Umschlingung um den Fußabschnitt (3) und den Unterschenkelabschnitt (2) der Bandage herumlegbar und unter Zugspannung mit seinem freien Ende (8) mittels eines Schnellverschlusses arretierbar ist.

## Claims

1. Ankle joint bandage made from elastic material having a lower leg section (2) and a foot section (3), in which in the heel region of the foot section (3), a pronation restraint (4) of tear-resistant and extension-resistant material is applied, which in the applied condition of the bandage can be stretched laterally essentially over the ankle joint extending in the direction of the lower leg section (2) and can be attached thereto, characterised in that the foot section (3) is designed to be essentially tubular and the lower leg section (2) is formed from two straps (9) which can be partly overlapped and can be made into a tubular shape by means of a rapid closure encompassing the bottom lower leg, and in that a stiffening insert (5), which extends from the heel region of the foot section (3) to the upper edge (6) of the lower leg section (2), lies medially in a textile pocket.

2. Ankle joint bandage according to claim 1, in which an elastic belt (7), which can be placed in a loop around the foot section (3) and the lower leg section (2) of the bandage and can be fixed with tensile stress at its free end (8) by means of a rapid closure, is applied medially in the heel region of the foot section (3).

## Revendications

1. Bandage de cheville réalisé en un matériau élastique et comprenant une section (2) située au niveau du bas de la jambe et une section (3) située au niveau du pied, et dans lequel dans la zone du talon de la section (3) située au niveau du pied est disposée une bride de pronation (4) formée d'un matériau résistant à l'arrachement et à la traction et qui, lorsque le bandage est appliqué, peut être tendue latéralement en s étendant essentiellement au-dessus de la cheville, en direction de la section (2) située au niveau de la partie inférieure de la jambe et peut y être fixée, caractérisé en ce que la section (3) située au niveau du pied est agencée avec une forme essentiellement tubulaire et la section (2) située au niveau de la partie inférieure de la jambe est formée de deux pattes (9) qui peuvent être amenées partiellement en chevauchement et qui peuvent être disposées au moyen d'un système de fermeture rapide de manière à prendre une forme tubulaire, qui entoure le bas de la partie inférieure de la jambe, et qu'un insert de renforcement (5) est disposé en position médiane dans une poche textile, cet insert s'étendant depuis la zone du talon de la section (3) située au niveau du pied jusqu'au bord supérieur (6) de la section (2) située au niveau de la partie inférieure de la jambe.

2. Bandage de cheville selon la revendication 1, dans lequel en position médiane dans la zone du talon de la section (3) située au niveau du pied est disposée une sangle élastique (7), qui peut être appliquée d'une manière enveloppante autour de la section (3) du bandage située au niveau du pied et de la section (2) du bandage située au niveau de la partie inférieure de la jambe et peut être fixée par son extrémité libre (8), sous une tension de traction, au moyen d'un système de fermeture rapide.
